# EUROPÄISCHE PATENTSCHRIFT

(11) **EP 1 219 591 B1**
(45) Veröffentlichungstag und Bekanntmachung des Hinweises auf die Patenterteilung: **31.03.2004**
(21) Anmeldenummer: 01129330.5
(22) Anmeldetag: 14.12.2001
(51) Int. Cl.: C07C 213/02, C07C 217/54, C07C 43/20, C07C 41/18

(54) **Verbessertes Verfahren zur Herstellung von 2-(4-Trifluormethoxyphenyl)ethylamin**
Process for preparing 2-(4-trifluoromethoxyphenyl)ethylamine
Procédé de préparation de la 2-(4-trifluoromethoxyphenyl)ethylamine

(30) Priorität: 27.12.2000 DE 10065442
(43) Veröffentlichungstag der Anmeldung: 03.07.2002
(73) Patentinhaber: Bayer Chemicals AG, 51368 Leverkusen (DE)
(72) Erfinder: Marhold, Albrecht, Dr., 51373 Leverkusen (DE); Müller, Peter, Dr., 51519 Odenthal (DE)

(56) Entgegenhaltungen:
- DE-A- 19 859 684
- US-A- 4 199 595
- US-A- 6 087 358
- ARAI M ET AL: "Gas-phase hydrogenation of nitriles by nickel on various supports" APPLIED CATALYSIS A: GENERAL, ELSEVIER SCIENCE, AMSTERDAM, NL, Bd. 148, Nr. 2, 2. Januar 1997 (1997-01-02), Seiten 405-413, XP004337993 ISSN: 0926-860X

## Beschreibung

Die vorliegende Erfindung betrifft ein verbessertes Verfahren zur Herstellung von 2-(4-Trifluormethoxyphenyl)-ethylamin aus Trifluormethoxybenzol.

2-(4-Trifluormethoxyphenyl)-ethylamin ist ein wichtiges Zwischenprodukt für die Herstellung von agrochemischen Wirkstoffen.

Es ist bereits bekannt, 2-(4-Trifluormethoxyphenyl)-ethylamin herzustellen, indem man 1-Brom-4-(trifluormethoxy)-benzol mit N-Vinyl-succinimid in einer Pd-katalysierten Heck-Reaktion umsetzt und anschließend das Umsetzungsprodukt einer Hydrierung und das Hydrierungsprodukt abschließend einer Hydrazinolyse unterwirft (s. DE-A 1 98 59 684 und DE-A 1 99 81 223). Für die Durchführung im technischen Maßstab ist dieses Verfahren weniger geeignet, weil die Heck-Reaktion und die anschließende Hydrierung Pd-Katalysatoren erfordern, die sehr teuer sind. Außerdem wird ein Succinylrest durch die Synthese geschleppt, der zum Erhalt des gewünschten Produkts wieder abgespaltet werden muss. Seine Handhabung verursacht großen Aufwand, auch im Hinblick auf seine Entsorgung, bzw. Rücküberführung in wiedereinsetzbares N-Vinyl-succinimid.

Es besteht daher noch ein Bedürfnis, nach einem effizienten, auch im technischen Maßstab gut durchzuführendes Verfahren zur Herstellung von 2-(4-Trifluormethoxyphenyl)-ethylamin.

Es ist auch bekannt, 4-Brommethyl- und 4-Chlormethyl-1-trifluormethoxybenzol ausgehend von 4-Methoxybenzoesäurechlorid in einem 4-stufigen Verfahren herzustellen. Hierbei wird 4-Methoxybenzoesäurechlorid zunächst zu 4-(Trichlormethoxy)-benzoesäurechlorid chloriert, das durch Behandlung mit Flusssäure in 4-(Trifluormethoxy)-benzoesäurefluorid überführt wird. Dieses Benzoesäurefluorid wird dann mit Lithiumaluminiumhydrid zu 4-(Trifluormethoxy)-benzylalkohol reduziert, den man schließlich mit Bromwasserstoffsäure in 4-Brommethyl-1-trifluormethoxybenzol oder durch Behandeln mit Thionylchlorid in 4-Chlormethyl-1-trifluormethoxybenzol überführt. Dieses Verfahren ist durch die 4-Stufigkeit und die erforderliche Handhabung von Lithiumaluminiumhydrid sehr aufwendig, so dass es für eine technische Anwendung auch weniger in Frage kommt. Ein Fachmann wird deshalb 4-Brommethyl- und 4-Chlormethyl-1-trifluormethoxybenzol nicht als Zwischenprodukte für eine effiziente Herstellung von 2-(4-Trifluormethoxyphenyl)-ethylamin in Betracht ziehen. Bisher wurden 4-Brommethyl- und 4-Chlormethyl-1-trifluormethoxybenzol nur für die Herstellung kleiner Mengen spezieller Wirkstoffe verwendet, die keine kommerzielle Bedeutung haben.

Es wurde nun ein Verfahren zur Herstellung von 2-(4-Trifluormethoxyphenyl)-ethylamin gefunden, das dadurch gekennzeichnet ist, dass man Trifluormethoxybenzol durch Halogenmethylierung in 4-Halogenmethyl-1-trifluormethoxy-benzol umwandelt, diese Verbindung durch einen Halogen-Cyan-Austausch in (4-Trifluormethoxyphenyl)-acetonitril überführt und dieses schließlich mit Wasserstoff in Gegenwart eines Nickelkatalysators reduziert.

Das als Einsatzmaterial für die erste Stufe benötigte Trifluormethoxybenzol ist ein großtechnisches Produkt und kommerziell erhältlich.

Die Umwandlung des Trifluormethoxybenzols zu einem 4-Halogenmethyl-1-trifluormethoxy-benzol, kann z.B. mit Formaldehyd und Bromwasserstoff, gegebenenfalls in Gegenwart einer Lewis-Säure und/oder einer Protonensäure als Katalysator durchgeführt werden. Man kann den Bromwasserstoff auch in situ aus einem Bromid, beispielsweise einem Alkalibromid, und einer starken Säure erzeugen. Es ist auch möglich, Natriumbromid in Gegenwart einer Lewis-Säure und/oder einer Protonensäure als Katalysator einzusetzen. Geeignete Lewis-Säuren sind beispielsweise Zinkchlorid und Aluminiumchlorid, geeignete Protonensäuren sind beispielsweise Phosphorsäure und Schwefelsäure. Die Menge der Lewis-Säure- bzw.

Protonensäure-Katalysatoren kann beispielsweise von 0,1 bis 100 Mol-%, bevorzugt 5 bis 10 Mol-%, bezogen auf eingesetztes Trifluormethoxybenzol betragen. Den Formaldehyd setzt man vorzugsweise in Form von Paraformaldehyd ein. Pro Mol Trifluormethoxybenzol kann man z.B. 1 bis 5 Mol, vorzugsweise 1,2 bis 3 Mol Formaldehyd einsetzen. Es ist vorteilhaft, das Bromid bzw. den Bromwasserstoff im Überschuss einzusetzen, beispielsweise pro Mol Trifluormethoxybenzol 1,2 bis 10 Mol, vorzugsweise 2 bis 5 Mol eines Bromids bzw. Bromwasserstoff. Als Lösungsmittel können z.B. Alkohole oder Carbonsäuren eingesetzt werden. Bevorzugt sind Methanol und Essigsäure. Die Reaktionstemperatur für diese Stufe kann in einem größeren Bereich variiert werden. Sie kann beispielsweise zwischen 0 und 100°C, bevorzugt zwischen 20 und 90°C betragen. Die Aufarbeitung des nach der Reaktion vorliegenden Reaktionsgemisches kann beispielsweise durch Eingießen in Eiswasser, Extraktion mit einem organischen Lösungsmittel, z.B. einem mit Wasser nicht mischbaren Ether, bevorzugt mit Methyl-tert.-butyl-ether, Einengen des organischen Extraktes und Destillation des Rückstandes erfolgen.

Auf völlig analoge Weise kann man aus Trifluormethoxybenzol 4-Chlormethyl-1-trifluormethoxy-benzol herstellen, wenn man statt Bromwasserstoff oder Bromiden Chlorwasserstoff oder Chloride, z.B. Natriumchlorid einsetzt.

Die zweite Stufe des erfindungsgemäßen Verfahrens, der Halogen-Cyan-Austausch, kann beispielsweise mit Natrium- oder Kaliumcyanid in wässrigem Alkohol durchgeführt werden. Vorzugsweise wird 4-Brommethyl- oder 4-Chlormethyl-1-trifluormethoxybenzol mit Natriumcyanid in einer Alkohol-Wasser-Mischung zu (4-Trifluormethoxyphenyl)-acetonitril umgesetzt. Die Alkohol/Wasser-Mischung kann z.B. Alkohol und Wasser im Volumen-Verhältnis von 2 bis 10 : 1, vorzugsweise 3 bis 8 : 1 enthalten. Geeignete Alkohole sind z.B. C₁-C₄-Alkylalkohole wie Methanol und Ethanol. Das Cyanid kann beispielsweise in einer Menge von 1 bis 10 Mol, bezogen auf 1 Mol 4-Brommethyl- bzw. 4-Chlormethyl-trifluormethoxy-benzol, eingesetzt werden. Vorzugsweise liegt diese Menge bei 1 bis 2 Mol. Die Reaktionstemperatur für diese Stufe kann in einem größeren Bereich variiert werden. Sie kann beispielsweise zwischen 0 und 100°C, bevorzugt zwischen 20 und 90°C liegen. Die Aufarbeitung des nach dem Halogen-Cyan-Austausch vorliegenden Reaktionsgemisches kann beispielsweise durchgeführt werden, indem man es in Wasser eingießt, mit einem mit Wasser nicht mischbaren organischen Lösungsmittel extrahiert, das organische Extrakt einengt und den Rückstand destilliert.

Für die dritte Stufe des erfindungsgemäßen Verfahrens, die Reduktion des (4-Trifluormethoxyphenyl)-acetonitrils zu 2-(4-Trifluormethoxyphenyl)-ethylamin mit Wasserstoff wird Raney-Nickel als Katalysator verwendet. Als Lösungsmittel können Ether wie Dioxan oder Tetrahydrofuran oder Alkohole wie Methanol oder Isopropanol verwendet werden. Um die Bildung von sekundären Aminen zu unterdrücken, kann es vorteilhaft sein, in Gegenwart von Ammoniak zu arbeiten. Pro Mol (4-Trifluormethoxyphenyl)acetonitril kann man z.B. 0,05 bis 0,5 Mol, bevorzugt 0,1 bis 0,3 Mol Nickel-Katalysator (berechnet als Metall) zusetzen. Der Wasserstoffdruck kann beispielsweise in Bereich von 50 bis 200 bar liegen und liegt vorzugsweise zwischen 90 und 140 bar. Die Reaktionstemperatur für diese Stufe kann in einem großen Bereich variiert werden, sie kann beispielsweise zwischen 50 und 200°C, bevorzugt zwischen 80 und 130°C liegen. Die Aufarbeitung des nach der Hydrierung vorliegenden Reaktionsgemisches kann beispielsweise durchgeführt werden, indem man zunächst den Katalysator abfiltriert und dann das Filtrat einer Destillation unterwirft.

Mit dem erfindungsgemäßen Verfahren lässt sich 2-(4-Trifluormethoxyphenyl)-ethylamin aus dem gut zugänglichen Trifluormethoxybenzol in einem technisch gut durchzuführenden Verfahren in guten Ausbeuten herstellen. Über alle Reaktionsstufen betrachtet liegt die Ausbeute deutlich höher als 40 % der Theorie. Die Handhabung von wieder abzuspaltenden Molekülteilen und die Verwendung teurer Pd-Katalysatoren wird vermieden.

### Beispiele

### Beispiel 1

### 4-Brommethyl-1-trifluormethoxy-benzol

300 g Trifluormethoxybenzol, 111 g Paraformaldehyd, 456 g Natriumbromid und 900 ml Eisessig werden vorgelegt und auf 90°C erwärmt. Dann wurde zur Freisetzung von Bromwasserstoff eine Mischung von 450 ml Eisessig und 678 g konzentrierter Schwefelsäure hinzugetropft. Nach beendeter Zugabe wurde das Reaktionsgemisch 20 Stunden bei 90°C gerührt. Anschließend wurde das Reaktionsgemisch auf 1000 g Eis gegossen und zweimal mit je 300 ml Methyl-tert.-butyl-ether extrahiert. Die vereinigten organischen Extrakte wurden mit 100 ml Wasser gewaschen und eingeengt. Der Rückstand wurde bei 20 hPa über eine Drehbandkolonne destilliert. Man erhielt 324,3 g 4-Brommethyl-1-trifluormethoxybenzol als farblose Flüssigkeit (60 % der Theorie) mit einem Sdp. von 82 bis 83°C.

### Beispiel 2

### 4-Chlormethyl-1-trifluormethoxy-benzol

200 g Trifluormethoxybenzol und 48 g Paraformaldehyd wurden in 500 ml Methanol gelöst und bei 60°C 7 Stunden lang trockenes Chlorwasserstoffgas eingeleitet. Die Abgase wurden über einen Rückflusskühler in wässriges Ammoniak geleitet. Anschließend wurde das Reaktionsgemisch auf 1000 g Eis gegossen und zweimal mit je 300 ml Methyl-tert.-butyl-ether extrahiert. Die vereinigten Extrakte wurden mit 100 ml Wasser, anschließend mit 100 ml Natriumhydrogencarbonatlösung gewaschen und dann eingeengt. Der Rückstand wurde bei 18 hPa über eine Drehbandkolonne destilliert. Man erhielt 158,8 g 4-Chlormethyl-1-trifluormethoxybenzol als farblose Flüssigkeit (65 % der Theorie) mit einem Sdp. von 71 bis 74°C.

### Beispiel 3

### (4-Trifluormethoxyphenyl)-acetonitril

150 g Ethanol, 28 ml Wasser und 31,5 g Natriumcyanid wurden vorgelegt und 127,5 g 4-Brommethyl-1-trifluormethoxybenzol zugegeben. Die Reaktionsmischung wurde 3,5 Stunden bei 90°C gerührt. Anschließend wurde das Reaktionsgemisch auf 250 ml Wasser gegeben und zweimal mit je 50 ml Dichlormethan extrahiert. Die vereinigten organischen Phasen wurden eingeengt und der Rückstand bei 18 hPa destilliert. Man erhielt 81,5 g (4-Trifluormethoxyphenyl)acetonitril als farblose Flüssigkeit (76,3 % der Theorie) mit einem Sdp. von 110 bis 112°C.

### Beispiel 4

### (4-Trifluormethoxyphenyl)-acetonitril

Es wurde gearbeitet wie in Beispiel 3, jedoch wurde die entsprechende Menge 4-Chlormethyl-1-trifluormethoxybenzol eingesetzt. Nach der Destillation erhielt man (4-Trifluormethoxyphenyl)-acetonitril als farblose Flüssigkeit in einer Ausbeute von 65 % der Theorie und mit einem Sdp. von 109 bis 111°C.

### Beispiel 5

### 2-(4-Trifluormethoxyphenyl)-ethylamin

650 ml Methanol, 260 g (4-Trifluormethoxyphenyl)-acetonitril und feuchtes Raney-Nickel in einer Menge entsprechend 0,39 Mol wurden in einem Autoklaven vorgelegt und 260 ml Ammoniak einkondensiert. Die Mischung wurde 2 Stunden bei 130°C und 140 bar Wasserstoffdruck hydriert. Anschließend wurde das Reaktionsgemisch bei Raumtemperatur entspannt und der Katalysator abfiltriert. Das Filtrat wurde bei 10 hPa destilliert. Man erhielt 224,5 g 2-(4-Trifluormethoxyphenyl)-ethylamin als farblose Flüssigkeit (86,7 % der Theorie) mit einem Sdp. von 84 bis 86°C.

## Patentansprüche

1. Verfahren zur Herstellung von 2-(4-Trifluormethoxyphenyl)-ethylamin, **dadurch gekennzeichnet, dass** man Trifluormethoxybenzol durch Halogenmethylierung in 4-Halogenmethyl-1-trifluormethoxybenzol umwandelt, diese Verbindung durch einen Halogen-Cyan-Austausch in (4-Trifluormethoxy)-acetonitril überführt und dieses schließlich mit Wasserstoff in Gegenwart **von Raney-Nickel** reduziert.

2. Verfahren nach Anspruch 1, **dadurch gekennzeichnet, dass** man die Halogenmethylierung mit Formaldehyd und a) Bromwasserstoff, gegebenenfalls in Gegenwart einer Lewis-Säure und/oder einer Protonensäure als Katalysator, oder b) durch Freisetzung von Bromwasserstoff aus einem Bromid und einer starken Säure oder c) Natriumbromid in Gegenwart einer Lewis-Säure und/oder einer Protonensäure als Katalysator durchführt.

3. Verfahren nach Ansprüchen 1 und 2, **dadurch gekennzeichnet, dass** man in die Halogenmethylierung 1 bis 5 Mol Formaldehyd und 1,2 bis 10 Mol Bromwasserstoff oder eines Bromids, bezogen auf 1 Mol Trifluormethoxybenzol einsetzt und diese Reaktionsstufe bei 0 bis 100°C durchführt.

4. Verfahren nach Ansprüchen 1 und 2, **dadurch gekennzeichnet, dass** man in die Halogenmethylierung mit 1 bis 5 Mol Formaldehyd und 1,2 bis 10 Mol Chlorwasserstoff oder eines Chlorids, bezogen auf 1 Mol Trifluormethoxybenzol einsetzt und diese Reaktionsstoffe bei 0 bis 100°C durchführt.

5. Verfahren nach Ansprüchen 1 bis 4, **dadurch gekennzeichnet, dass** man den Halogen-Cyan-Austausch mit Natrium- oder Kaliumcyanid in wässrigem Alkohol bei Temperaturen im Bereich 0 bis 100°C durchführt.

6. Verfahren nach Ansprüchen 1 bis 5, **dadurch gekennzeichnet, dass** man als Nickelkatalysator Raney-Nickel in einer Menge von 0,05 bis 0,5 Mol (berechnet als Metall) pro Mol (4-Trifluormethoxyphenyl)-acetonitril einsetzt.

7. Verfahren nach Ansprüchen 1 bis 6, **dadurch gekennzeichnet, dass** man die Reduktion bei Wasserstoffdrucken im Bereich von 50 bis 200 bar und bei Temperaturen im Bereich 50 bis 200°C durchführt.

## Claims

1. Process for preparing 2-(4-trifluoromethoxyphenyl)-ethylamine, **characterized in that** trifluoromethoxybenzene is converted by halomethylation into 4-halogenomethyl-1-trifluoromethoxybenzene, this compound is converted by halogen-cyano exchange into (4-trifluoromethoxy)-acetonitrile and this compound is finally reduced using hydrogen in the presence of Raney nickel.

2. Process according to Claim 1, **characterized in that** the halomethylation is carried out using formaldehyde and a) hydrogen bromide, if appropriate in the presence of a Lewis acid and/or a protic acid as catalyst, or b) by releasing hydrogen bromide from a bromide and a strong acid or c) using sodium bromide in the presence of a Lewis acid and/or a protic acid as catalyst.

3. Process according to Claim 1 or 2, **characterized in that** in the halomethylation from 1 to 5 mol of formaldehyde and from 1.2 to 10 mol of hydrogen bromide or a bromide are employed per mole of trifluoromethoxybenzene, and this reaction step is carried out at from 0 to 100°C.

4. Process according to Claim 1 or 2, **characterized in that** in the halomethylation from 1 to 5 mol of formaldehyde and from 1.2 to 10 mol of hydrogen chloride or a chloride are employed per mole of trifluoromethoxybenzene, and this reaction step is carried out at from 0 to 100°C.

5. Process according to any of Claims 1 to 4, **characterized in that** the halogen-cyano exchange is carried out using sodium cyanide or potassium cyanide in aqueous alcohol at temperatures in the range from 0 to 100°C.

6. Process according to any of Claims 1 to 5, **characterized in that** the nickel catalyst used is Raney nickel in an amount of from 0.05 to 0.5 mol (calculated as metal) per mole of (4-trifluoromethoxyphenyl)-acetonitrile.

7. Process according to any of Claims 1 to 6, **characterized in that** the reduction is carried out at hydrogen pressures in the range from 50 to 200 bar and at temperatures in the range from 50 to 200°C.

## Revendications

1. Procédé pour la préparation de 2-(4-trifluorométhoxyphényl)-éthylamine, **caractérisé en ce que** l'on transforme du trifluorométhoxybenzène par halogénométhylation en 4-halogénométhyl-1-trifluoro-méthoxybenzène, on convertit ce composé par un échange halogène-cyano en (4-trifluorométhoxy)-acétonitrile et on réduit finalement celui-ci avec de l'hydrogène en présence de nickel de Raney.

2. Procédé selon la revendication 1, **caractérisé en ce que** l'on réalise l'halogénométhylation avec du formaldéhyde et a) de l'acide bromhydrique, éventuellement en présence d'un acide de Lewis et/ou d'un acide protonique comme catalyseur, ou b) par libération d'acide bromhydrique à partir d'un bromure et d'un acide fort ou c) du bromure de sodium en présence d'un acide de Lewis et/ou d'un acide protonique comme catalyseur.

3. Procédé selon les revendications 1 et 2, **caractérisé en ce que** l'on utilise dans l'halogénométhylation de 1 à 5 mol de formaldéhyde et de 1,2 à 10 mol d'acide bromhydrique ou d'un bromure, rapporté à 1 mol de trifluorométhoxybenzène et on réalise cette étape de réaction à de 0 à 100°C.

4. Procédé selon les revendications 1 à 2, **caractérisé en ce que** l'on utilise dans l'halogénométhylation de 1 à 5 mol de formaldéhyde et de 1,2 à 10 mol d'acide chlorhydrique ou d'un chlorure, rapporté à 1 mol de trifluorométhoxybenzène et on réalise cette étape de réaction à de 0 à 100°C.

5. Procédé selon les revendications 1 à 4, **caractérisé en ce que** l'on réalise l'échange halogène-cyano avec du cyanure de sodium ou de potassium dans un alcool aqueux à des températures dans le domaine de 0 à 100°C.

6. Procédé selon les revendications 1 à 5, **caractérisé en ce que** l'on utilise comme catalyseur de nickel du nickel de Raney dans une quantité de 0,05 à 0,5 mol (calculé comme métal) par mole de (4-trifluorométhoxyphényl)-acétonitrile.

7. Procédé selon les revendications 1 à 6, **caractérisé en ce que** l'on réalise la réduction à des pressions d'hydrogène dans le domaine de 50 à 200 bars et à des températures dans le domaine de 50 à 200°C.
